# EUROPEAN PATENT APPLICATION

(11) **EP 2 177 604 A1**
(43) Date of publication of application: **21.04.2010**
(21) Application number: 08166235.5
(22) Date of filing: 09.10.2008
(51) Int. Cl.: C12N 9/02, C12N 15/81

(54) **Methods for identifying potential CYP4 metabolites, inhibitors or prodrugs**

(71) Applicant: PomBio Tech GmbH, 66123 Saarbrücken (DE)
(72) Inventor: The designation of the inventor has not yet been filed
(74) Representative: Fleuchaus, Andrea

(57) **Abstract**

The present invention relates to an improved yeast based whole cell expression systems for the expression of class 4 cytochrome P450s. Further, the present invention relates to the disease related expression of CYP4s, such as CYP4Z1 as well as methods to identify potential CYP4 metabolites, inhibitors or prodrugs.

## Description

The present invention relates to a yeast based whole-cell expression system for the expression of class 4 cytochrome P450s. Further, the present invention relates to the disease related expression of CYP4s, such as CYP4Z1 as well as methods to identify potential CYP4 metabolites, inhibitors or prodrugs.

Cytochrome P450 enzymes represent a superfamily of b-type heme proteins ubiquitously distributed in different organisms from all biological kingdoms. These membrane bound external mo-nooxygenases play an important role in the metabolism of many distinct drugs, carcinogens, alkaloids, pesticides and other important xenobiotics as well as in a variety of physiologically relevant processes e.g. for members of class II (CYP2) steroid hormone biosynthesis (Bernhardt, R., (2006), J. Biotechnology 124, 128-145). In the case of the drug metabolizing microsomal cytochromes P450 are involved, which mainly belong to the P450 family Class III (CYP3 family). Members of class IV cytochrome P450s (CYP4 family) are known to mainly hydroxylate saturated and unsaturated fatty acids.

The preferred reaction catalyzed by members of the CYP4 family is the hydroxylation of fatty acids, such as the ω-hydroxylation. This reaction mainly serves the purpose to avoid the excessive accumulation of free fatty acids, which after formation of dicarboylic acids, can be excreted. Moreover, CYP 4 family members are also involved in the degradation of signalling molecules such as prostanoids and leukotrienes, and further participate in the production of bioactive metabolites from arachidonic acid such as 20-hydroxyeicosatetraenoic acid, which plays a role in the regulation of blood pressure.

However, some members of this family are still orphan P450 i.e. P450s that could not be associated with a specific reaction or physiological function. This is particularly true e.g. CYP4A22, CYP4F11, CYP4F22, CYP4V2, CYP4X1 and CYP4Z1, which are therefore considered to belong to the group of orphan P450s [Stark,K. & Guengerich, F.P. (2007), Drug Metabolism Reviews 39, 627-637).

Currently, efforts are being undertaken to assess the function of these P450s by trying to purify these proteins after bacterial expression in *E. coli.* However, since P450s are membrane bound proteins their expression and the subsequent purification has turned out to be rather difficult rendering only small amounts of protein. In this context it is noteworthy to mention that the group of Guengerich has very recently succeeded in purifying human CYP4X1 and in identifying new substrates for this "ex-orphan P450" (Stark, K., et al. (2008), Febs Journal 275, 3706-3717).

One interesting orphan P450s is human CYP4Z1. The mRNA expression pattern of this human P450, localized on chromosome 1p33, revealed an significant expression in mammary tissue, breast carcinoma cells as well as primary ovarian cancer cells, whereas it is almost undetectable in other human tissues (Rieger, M.A. et al. (2004), Cancer Research 64, 2357-2364; Downie, D. et al. (2005), Clinical Cancer Research 11, 7369-7375).

Although the tissue specific expression and particularly the cancer specific expression of these cytochromes are known, there is still a need for further characterisation of these disease and cancer related cytochrome P450's. Of particular interest is to understand their physiological functionality and effects as well as the consequences of disease causing deregulation. Only with a detailed understanding of the physiological functionality it is possible to develop pharmaceuticals inhibitors, which specifically overcome the disease causing deregulation or are able to compensate his malfunction.

In summary, there is a serious need to establish methods, which improve and assist scientific research regarding the characterisation of physiological functionality as well as deregulation of cytochrome P450. This task is particularly complicated, since the cytochrome P450's besides having the same name are very complex and diverse.

The superfamily of cytochrome P450 presently comprises more than 5000 different genes, which have been cloned, but not all yet characterised. These enzymes catalyse a variety of interesting chemical reactions, such as monooxygenase, oxidase and peroxidase reaction, and further reactions, such as, and this is only meant to be an excerpt, carbon hydroxylation, heteroatomoxygenation, dealkylation, sulphoxidation, epoxidation, aromatic hydroxylation, deamination reduction or dehalogenation (Guengerich, F.P. (2001a), Chem. Res. Toxicol 14, 611-650, (2001b) Mutat. Res. 488, 195-202). Their substrates include fatty acids, steroids, prostaglandins, most drugs or xenobiotics, organic solvents, carcinogens, pestisites, insecticides etc.

Beside all this complexity most cytochrome P450's seem to follow a general reaction cycle of a stepwise activation of molecular oxygen and an interaction with an electron donating system. While this general reaction appears to be consistent, the cytochromes are quite distinct in their location and regarding the need of interaction with the electron donating systems. Thus, it is still a challenge to establish functional expression and test systems.

The literature provides proof that many expression systems based on bacterial, yeast, insect or mammalian cell systems have been established. Using these systems the scientific community has learned a lot about the protein structure, protein folding, the active domains, the binding sites for the substrates etc. Most of these studies have been performed using enzyme preparations or purified enzyme. Nevertheless it is still a challenge to establish assays, which allow studying the various metabolites of the different P450's reactions.

For establishing the expression system for functional CYP4Z1 the present invention provides recombinant fission yeast. The fission yeast is selected from the group comprising *Schizosaccharomycetes*, such as *Schizosaccharomyces pombe* as well as strains derived from *Sc. pombe* and related yeast strains.

Lindner has scientifically described *Schizosaccharomyces pombe* in 1893. Its genomic sequence was published in 2002 (Wood et al., (2002), Nature 415, 871-880). Interestingly, *Schizosaccharomyces pombe*, although being yeast, from an evolutionary point of view is very distinct from the well-known bakers yeast *Saccharomyces cerevisiae.*

The term "related fission yeast strains" defines herein yeast strains, which have the same functional characteristics as *Schizosaccharomyces pombe.* Furthermore, strains derived from *Schizosaccharomyces pombe* refer to recombinant as well as conventionally or molecularly modified strain of this yeast, whereby the modifications are insertions, exchanges, additions or deletions of genomic sequence, which may show in the phenotype.

The fission yeast according to the invention has been transformed to express the nucleic acid sequence encoding CYP4Z1 or functional variants thereof The inventors found that this expression system is already functional without the need of coexpression of an electron donating system. This is unexpected because it is known that all human cytochromes normally need an electron donating system, such as a cytochrome P450 reductase.

However, according to a further embodiment the expression system of the present invention encoding CYP4Z1 sequence may be further modified to coexpress a nucleic acid sequence encoding the human NADPH-dependent cytochrome P450 reductase (CPR) or functional variants thereof in order to, optionally, increase the CYP4Z1-dependent activity. The fission yeast-based expression system according to the present invention is therefore capable of expressing functional human CYP4 and/or functional CPR.

The most challenging problem regarding this expression system of the present invention is the fact that the expression of a CYP4Z1 and to an even larger extent the expression of CPR causes growth retardation in fission yeast. The growth retardation observed was in a range of about 50% to 150%.

To compensate for this the inventors used various techniques to modify the expression level of CYP4 and/or CPR as well as the expressed ratio of CYP4 versus CPR.

To this end, a fission yeast strain, preferably *S. pombe*, is transformed with either integrative or autosomally replicating vectors, which carry under the regulatory control of at least one promoter element the nucleic acid sequence encoding human CYP4 and, optional, a functional CPR.

According to a further embodiment the promoter elements used in the present invention are of different strength or are inducible promoter elements, which can be induced, switched on and off according to the needs of the experiment and the expression system.

In cases where the expression system according to the invention expresses a human CYP4 and a CPR the promoter elements should be chosen in a way to influence the ratio of the expression of functional CYP4 versus CPR. Ideally, the ratio is larger than 1:1, such as 1.1:1, 1,5:1, 2:1, 3:1, 4:1, 5:1, 6:1, 7:1, 8:1, 9:1, 10:1 and even up to 50:1. The positive effect of such CYP4 dominated ratio on the whole cell bioconversion system is highly surprising, particularly since the state of the art regularly disclosed a correlation of an increased CPR expression with a increased cytochrome activity. While the present invention shows that the best results are obtained with a strong CYP4 expression combined with a moderate or even low CPR expression. Consistently with this the coexpression system for CYP4Z1 and CPR enhanced the substrate conversion rate.

The - according to the present invention - improved functional expression system is therefore clearly providing advantages over the known expression systems and, thus, improves the methods to study physiological characteristics as well as the method to identify substances potential interfering with cytochromes of family 4, and particularly CYP4Z1.

In the context of the present invention the following definitions shall be relevant.

The term "nucleic acid sequence" as used herein refers to an oligonucleotide, nucleotide, or polynucleotide, and fragments or portions thereof, and to DNA, cDNA or RNA of genomic or synthetic origin, which may be single- or double-stranded, and represent the sense or antisense strand.

The term "CYP4", as used herein, refers to the amino acid sequences of cytochrome P450 enzymes of the family 4 from any microbial, plant or animal species, particularly mammalian, including bovine, ovine, porcine, murine, equine, simian, and preferably human, from any source whether natural, synthetic, semi-synthetic or recombinant. Particularly, the term includes the previously identified and described members of the human class IV cytochrome P450 family, such as CYP 4A22, 4F11, 4F22, 4V2, 4X1 and 4Z1. "NADPH-dependent cytochrome P450 reductase" or "CPR", as used herein, refers to the amino acid sequences of substantially purified enzyme, cytochrome P450 reductase, obtained from any microbial, plant or animal species, particularly mammalian, including human, from any source whether natural, synthetic, semi-synthetic or recombinant. CPR is an membrane bound enzyme and catalyzes an NADPH-dependent transfer of electrons to cytochrome P450 enzymes. Particularly, the term as used herein includes the previously identified and described human CPR.

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule.

A "variant", as used herein, refers either to a nucleic or amino acid sequence that is altered by one or more nucleotides or amino acids. The term "variant" particularly includes the nucleic acid sequences, which have been optimized for strain or organism specific codon usage. Additionally, the term encloses nucleic acid sequences, which have nucleotide exchanges due the degeneration of the genetic code or more interestingly due to individual polymorphisms.

Further, the "variant" of an amino acid sequence may have conservative amino acid changes, wherein a substituted amino acid has similar structural or chemical properties, e.g., replacement of leucine with isoleucine. More rarely, a variant may have non-conservative changes, e.g., replacement of a glycine with a tryptophan. Similar minor variations may also include amino acid deletions or insertions, or both. Guidance in determining which amino acid residues may be substituted, inserted, or deleted without abolishing biological or immunological activity may be found using computer programs well known in the art.

Further, the term "functional variant" is understood herein as a variant amino acid sequence, a peptide derived from the original amino acid sequence or a functional domain of the original amino acid sequence, which still shows the same enzymatic, biological or immunological activity as the original protein, peptide or active domain. A variant is still considered a "functional variant" if the amount of activity varies.

The term "transformed", as defined herein, describes a process (transformation) by which exogenous DNA enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but are not limited to, viral infection, electroporation, lipofection, and particle bombardment. Such "transformed" cells include stably transformed cells in which the inserted DNA is capable of replication either as an autonomously replicating plasmid or as part of the host chromosome. They also include cells, which transiently express the inserted DNA or RNA for limited periods of time.

In still another embodiment, using the orphan P450, CYP4Z1, the inventors have provided and a method to characterise CYP4 enzymes e.g. the CYP4Z1 together with their substrates and inhibitors. The expression system according to said further embodiment expresses the functional cytochrome P450 CYP4Z1 as well as the human electron donating system, namely the cytochrome P450-reductase, CPR.

For this, they firstly established a method to identify the natural and physiological substrate of the CYP4 e.g. CYP4Z1, which so far were unknown. This method involves the use of a biotransformation assay employing the whole cell expression system for CYP4, e.g. CYP4Z1 as well as analytic techniques such as GC-MS and HPLC.

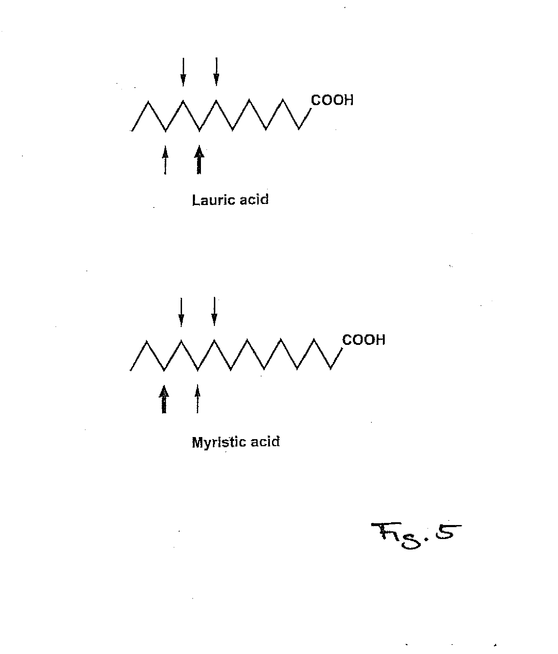

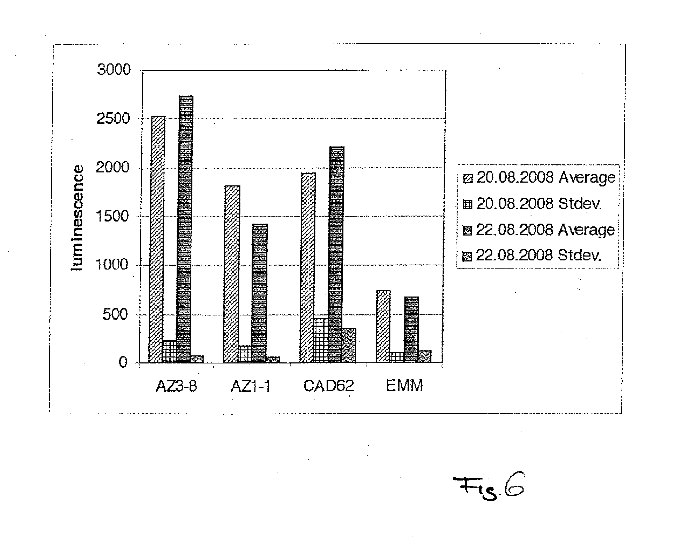

As a result (for details see Examples) lauric and myristic acid were identified as natural substrate of CYP4Z1. The assay system revealed that CYP4Z1 is able to hydroxylate these substrates at different positions (Fig 5). Based on the MS fragmentation analysis lauric acid is predominantly ω-4-hydroxylated whereas myristic acid is mainly hydroxylated at position ω-2. Besides these main products they were also able to detect other hydroxylated fatty acids namely ω-2-, ω-3-, ω-5-hydroxylated lauric acid as well as ω-3-, ω-4-, ω-5-hydroxlated myristic acid.

This finding might reflect that human CYP4Z1 does not selectively hydroxylate its substrates. Considering these results it can further be assumed that human CYP4Z1 preferentially hydroxylates medium-long chain saturated fatty acids (C12 and C14) whereas fatty acids containing longer carbon backbones seem not to be typical CYP4Z1 substrates.

The physiological role of the hydroxy fatty acids identified as CYP4Z1 metabolites has not been investigated so far. The fact that myristic acid is hydroxylated by human CYP4Z1 opens many questions since protein myristolation is an often used posttranslational modification of proteins and plays a role in G-protein mediated signalling cascades.

Considering that CYP4Z1 is overexpressed in breast cancer tissue it might be possible that due to the action of this cytochrome the amount of free myristic acid is significantly reduced blocking G-protein signalling cascades. Moreover, it is known that 2-hydroxymyristic acid efficiently inhibits the myristoyl-CoA:protein N-myristoyltransferase, the enzyme that catalyzes protein N-myristoylation. Taking this into account, it could be possible that the discovered CYP4Z1 myristic acid metabolites also inhibit this enzyme. Thus, due to the action of CYP4Z1 either the myristyltransferase may be inhibited or that due to a reduced availability of myristic acid less proteins are myristolated.

The possible role of lauric acid or its metabolites in cancerogenesis is not yet understood. So far it is known that lauric acid is a potent anti-microbial agent, which is also present in human breast tissue and milk. Previous studies have carefully postulated that the administration of lauric acid rich products, e.g. coconut oil, could help to prevent tumour formation or might even exhibit a tumour suppressing function (Salerno, J.W. et al. (1991), Anticancer Research 11, 209-215).

According to a further embodiment the present invention also provides a method to identify CYP4Z1-dependent tumour markers. This method is particularly of interest in the context of ovarian cancer, for which Downi et al. (2005, Clin. Canc. Res. 11, 7369-7375) had shown a correlation between mortality and CYP4Z1 expression level. The method according the present invention allows comparative studies on the CYP4Z1-dependenr metabolites of tumor cell specific, endogenous substances and, thus, can be used to identify a potential tumour marker.

According to a further embodiment of the present invention a method to identify inhibitors of the formation of CYP4-dependent and particularly CYP4Z1-dependent metabolites is provided.

For this, a yeast based whole cell bioconversion system expressing CYP4, e.g. CYP4Z1, and optionally CPR for converting lauric acid and/or myristic acid is established. In a second step a parallel whole cell bioconversion system is established to which test substances are added. In a comparative analysis of the first and the second bioconversion system the capability of the test substances is analysed for inhibition of the formation of lauric and/or myristic acid metabolites.

It is important to note that with this method - for the first time possible - to identify substances, which are capable to inhibit the conversion of one, but not the other of the natural substrates of the orphan CYP4s, such as CYP4Z1.

In one further embodiment the method to identify inhibitors of the formation of CYP4-dependent and particularly CYP4Z1-dependent metabolites uses as substrate for conversion a detectable dye, which is metabolised by the CYP4 and allows thereby analytic detection of enzyme activity or enzyme inhibition. According to one embodiment the conversion substrate is Luciferin or Luciferin-6' pentafluorobenzyl-ether.

The above mentioned test systems allow not only to analyse enzyme activity, but also to test already known substances or drugs for their side effects, which might affect the prolactive activity of the female breast or, more important, which might modulate the breast or ovarian cancer risk of a patient treated with such drugs.

According to still a further embodiment these test systems allow to identify chemical substances and prodrugs, which are activated specifically by CYP4, such as CYP4Z1 and which generate active metabolites useful as therapeutic composition e.g. in an approach to tissue specific treatment of breast and ovarian cancer.

For the general understanding the term "prodrug" as used herein refers to a pharmacological substance (drug) that is administered in an inactive (or significantly less active) form. Once administered, the prodrug is metabolised *in vivo* into an active metabolite. The rationale behind the use of a prodrug is generally for absorption, distribution, metabolism, and excretion optimization. Prodrugs are usually designed to improve bioavailability. Additionally, the use of a prodrug strategy increases the selectivity of the drug for its intended target. An example of this can be seen in many chemotherapy treatments, in which the reduction of adverse effects is always of paramount importance.

According to still a further embodiment the expression system according the invention can also be used to generate or manufacture according to know standard procedures an enzyme preparation having the CYP4Z1 specific activity.

### Short description of the Figures

**Figure 1****: *S. pombe* strain growth curves.**
   5*10⁶ cells were cultured in 100 mL Edinburgh minimal media. 1 mL samples were collected at different time points and centrifuged. Pellets were then dried and weighed.
**Figure 2****: Total ion chromatograms for different fatty acid containing samples.**
   Whole-cell biotransformation of strain AZ3 with two saturated fatty acids. Shown are the total ion chromatograms that were obtained after extraction and analysis of cultures that contained the substrates lauric acid (A) or myristic acid (B), resp.. In each biotransformation four product peaks were observed that were absent in control experiments with the parental strain CAD62.
**Figure 3A and3B: MS spectra of the identified OH-fatty acids**
   Displayed are the MS spectra of the identified derivatized products obtained using the strain AZ3 as well as the structure of each metabolite. (A) lauric acid and (B) myristic acid metabolites.

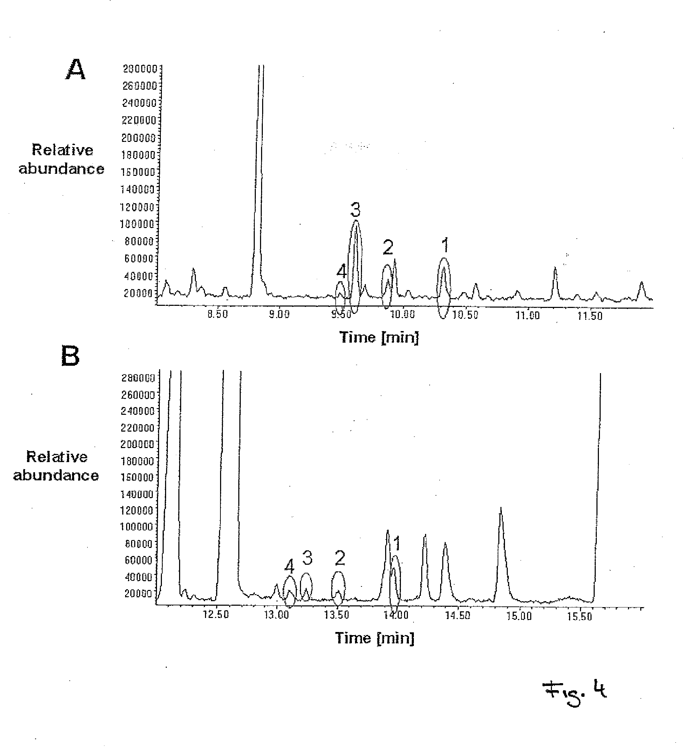

**Figure 4****: Fatty acid hydroxylation pattern of CYP4Z1.**
   The observed hydroxylation positions of lauric acid and myristic acid are indicated, with bold arrows indicating the preferred reaction sites.
**Figure 5****: The diagram shows the luminescence signal useful in a drug screening assay**
   Determination of the luminescence signal from AZ3-8, AZ1-1, CAD62 (EMM as background) after four hours incubation with luciferin-PFBE from two independent measurements. It is shown that the strain AZ3-8 has the higher signal. Both bars with white background are from the 1st measurement. Bars with grey background are from the 2nd measurement.

### Examples:

### 1. Establishing of a yeast-based cell expression system expressing CYP4Z1

The human CYP4Z1 cDNA and the human cytochrome P450 reductase (CPR) sequences were taken from UniProtKB/Swiss-Prot (entries Q86W10 and P16435), codon optimized by Entelechon GmbH (Regensburg, Germany) for usage in fission yeast and delivered as pPCR-ScriptAmpSK(+) (Stratagene, La Jolla, CA, USA) clones. All cDNAs were amplified using standard molecular biology techniques. Purified CYP4Z1 cDNA constructs were subsequently cloned via NdeI and BamH1 restriction (Stratagene) into the expression vector pCAD1, to yield pCAD1-4Z1 as well as into the autosomal expression vector pREP yielding pREP-4Z1. The human CPR gene was inserted into pCADI via the Nde1/Bamh1 cloning site of this vector leading to the generation of pCAD1-CPR. Transformation of the fission yeast strains was performed using a protocol for cryopreserved, competent fission yeast cells. Fission yeast strain NCYC2036 with genotype h ura4.d118 was used as parental strain for the creation of AZ1, a fission yeast strain containing the cDNA of human CYP4Z1 integrated into the *S.pombe* genome, and CAD62, which integrated the human CPR gene. CAD62 served subsequently as parental strain for the generation of the fission yeast strain AZ3, which contains the human CPR gene integrated into the fission yeast genome and an autosomal plasmid expressing human CYP4Z1. Transformants were either streaked on EMM + leucine + thiamine (pCAD1-4Z1 and pCAD1-CPR transformants) or on EMM + thiamine (pREP- 4Z1 transformants) dishes and grown for approximately 72 h yielding colonies of the respective strains.

### 2. Substrate conversion system

Possible substrates of human CYP4Z1 were tested by incubating the fission yeast strains AZ1 and AZ3 as well as the control strains NCYC2036 and CAD 62 with 1 mM substrate. Different steroids, vitamin D, ebastine as well as saturated and unsaturated fatty acids were tested as possible CYP4Z1 substrates. Approximately 2*10⁸ cells/mL were used per assay. Cells were grown in EMM medium containing 100 g/L glucose as well as the required supplements (leucine for AZ1 and CAD 62). Incubation was carried out either in 96 well microtiter plates or in 250 mL Erlenmeyer flasks with a cellulose stopper at a shaking velocity of 150 rpm for 24 h at 30°C.

Samples containing steroids as possible substrates were extracted with ethylacetate, evaporated and analyzed via HPLC (Alliance, Waters, USA) using a RP-18 column (Licrosphere 100, 5µM, Merck, Darmstadt) or a RP-8 column (Licrosphere 100, 5µM, Merck, Darmstadt). The column temperature was kept constant at 40°C. The mobile phase used for each sample is outlined in Suppl. Table. 1. When available, positive control reactions were carried out using strains expressing a P450 that is known to metabolize the respective substrate.

Fatty acid conversions were analyzed via GC-MS as previously described. Fatty acid extraction from *S.pombe* cells was performed using the Bligh and Dyer method.. Extracted samples were then evaporated to dryness, dissolved in a mixture of 500 µl methanol/toluene/sulphuric acid (50:50:2 V/V) and incubated at 55 °C overnight. 400µl of a 0.5 M NH4CO3, 2 M KCl solution were added to the samples which were then centrifuged at RT, transferred to a GC vial and derivatised with 25 µl N-Methyl-N-(trimethylsilyl)trifluoroacetamide (37 °C for 1 hour). Fatty acid analysis was performed on an Agilent 6890N gas chromatograph coupled to an Agilent 5973N mass selective detector and equipped with a non-polar J&W DB-5HT capillary column, as described before.

### 3. Characterisation of the yeast based expression system

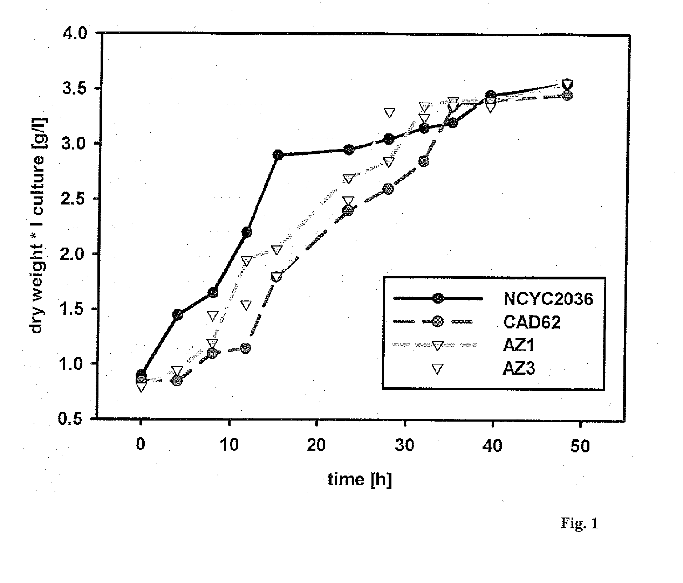

The goal of this work was to create and establish a system that can be used to identify substrates for the orphan cytochrome CYP4Z1. In a first analysis we could show that the co-expression of human CPR enhances the P450 dependent substrate conversion. Therefore, we created a strain that is capable of expressing both, CYP4Z1 and CPR (AZ3), as well as a strain that solely expresses CYP4Z1 (AZ1) (see Example 1). Both CYP4Z1 expressing strains revealed no morphological changes compared to the parental strains. In order to investigate whether the expressed cytochrome alters the growth parameters of fission yeast we performed growth assays by measuring the dry weight at different time points Figure 1 shows the growth curves obtained for the two 4Z1 expressing strains were not significantly altered compared to the parental strains indicating that the expression of CYP4Z1 does not dramatically alter the growth behaviour. All transformed strains displayed a decreased growth behaviour compared with the untransformed parental strain NCYC2036. The most pronounced effect can be seen in CAD62, which solely expresses human CPR. Preliminary data indicates that these effects are at least in part caused by a slightly increased ROS production being a side effect of the expression of CYP4Z1 and CPR in *S. pombe.*

### 4. Substrate identification assay

In order to identify human CYP4Z1 catalyzed reactions we decided to analyze different substrates that could be physiologically relevant. Since it is known that human CYP4Z1 is overexpressed in breast cancer tissue it seemed logical to test substrates that might be involved in cancerogenesis or that are abundant in this tissue. Steroid hormones are known to be involved in the generation and progression of breast as well as prostate cancer. Therefore, it was decided to test a set of different steroid hormones as possible CYP4Z1 substrates. Even if family 4 cytochromes are not typical steroid hydroxylating enzymes they have evolved from cholesterol oxidizing CYPs. This finding probably indicates that members of this family are still capable of oxidizing cholesterol or to hydroxylate steroid hormones. However, the results obtained from our biotransformation assays revealed no metabolite formation when using either cholesterol or several steroid hormones as possible CYP4Z1 substrates. Moreover, vitamin D2 and D3, which are both known to decrease the breast cancer risk and in contrast to the overexpression of CYP4Z1 contribute to a good prognosis for breast cancer patients when present in high quantities, as well as ebastine, a known human CYP4F12 substrate, were also not metabolized by human CYP4Z1.

Furthermore, since most of the members of this cytochrome family are known fatty-acid hydroxylases it was straightforward to test whether human CYP4Z1 is capable of hydroxylating different fatty acids. In order to pay tribute to the physiological environment of CYP4Z1 it was decided to focus on the fatty acid composition of human breast tissue. Taking this into consideration, it was tested a set of saturated fatty acids as well as the two major unsaturated fatty acids present in breast tissue (oleic and linoleic acid), as possible CYP4Z1 substrates. Since the amount of arachidonic acid, a known CYP family 4 substrate, is relatively low in human breast tissue it was decided only to test whether the arachidonic acid precursor linoleic acid is a CYP4Z1 substrate or not.

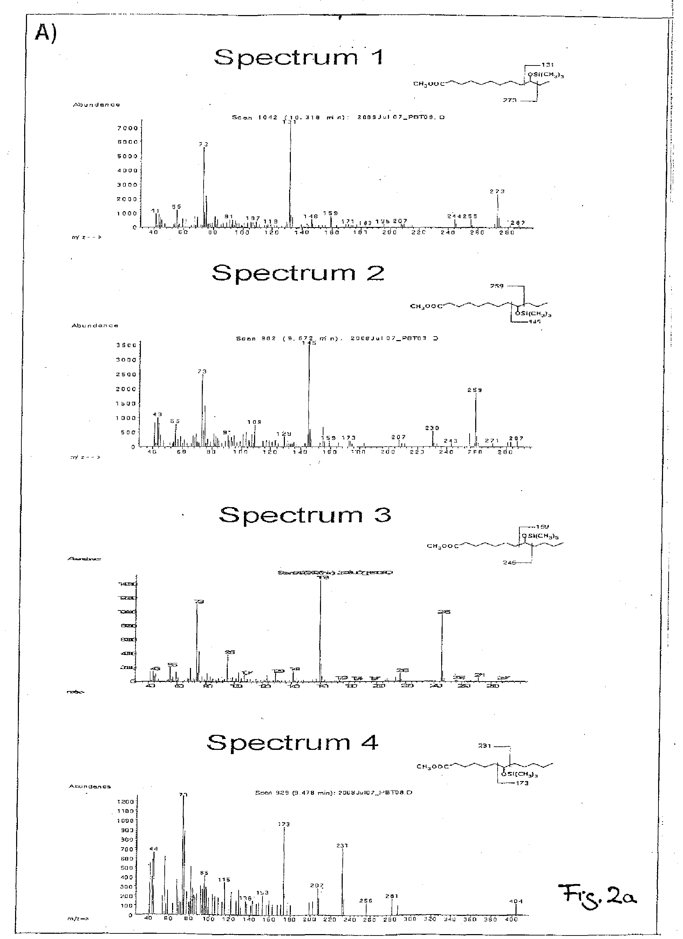

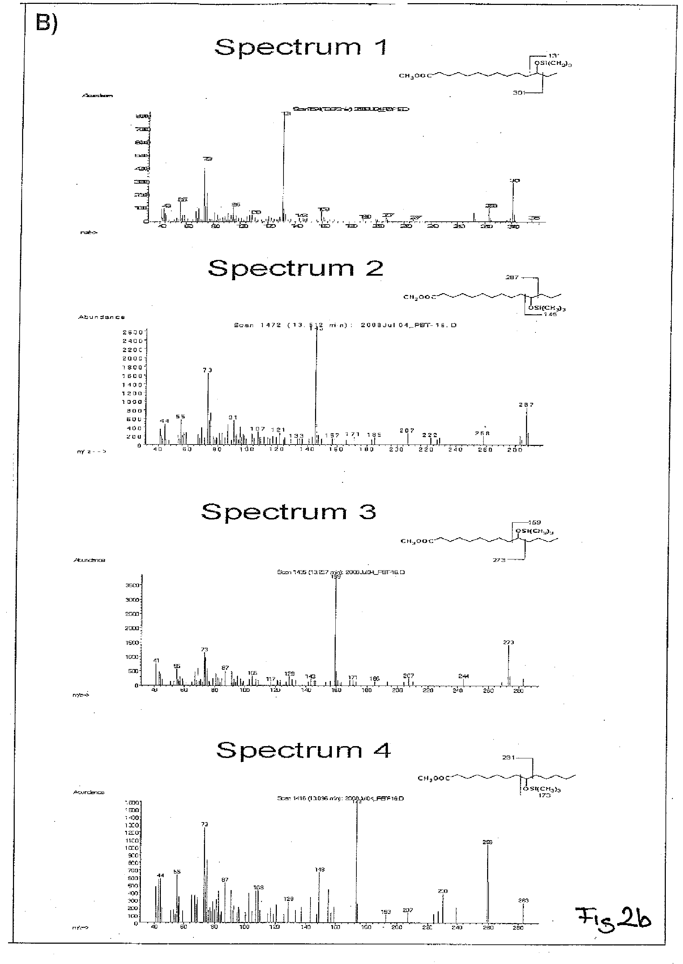

The results obtained from these studies clearly showed that human CYP4Z1 is able to hydroxylate lauric as well as myristic acid at different positions. Figure 2 shows samples generated using our CYP4Z1 fission strains contained fatty acid metabolites whereas these metabolites where not present in the control samples.

### 5. Luminescence assay

For simplified application of the method for identification of CYP4Z1 modulators a luminescence assay was developed.

For this, a standardised substrate was used in the reference assay. As substrate the dye luciferin was used, which will be dealkylated by CYP4Z1. The metabolite can be measured after addition of the detection reagent.

In brief, fission yeast-cells of the strains AZ1-1, AZ3-8 and CAD62 are cultured in a 10ml pre-culture with EMM-medium for approx. 24h. A main-culture with the amount of 100ml EMM-medium is then inoculated with the pre-culture and grown for 24h. After one day, the fission yeast culture's cell-density is determined with the aid of a Neubauer-counting chamber and the amount of cell-suspension that corresponds to a cell-amount of 1x107 cells is centrifuged (5000g, 5min, room temperature) and resuspended in 200µl of EMM-medium. The measurements are accomplished in form of triple values for each strain. To the amount of 200µl cell suspension for each sample, 5µl of the substrate Luciferin-PFBE (luciferin-6' pentafluorobenzyl-ether; promega) is added and the samples are incubated horizontally for four hours at 30°C and 150rpm (in 1.5ml Eppendorf tubes). After the incubation, the cells are centrifuged at 5000g for 5min at room temperature. In white NUNC96-well plates, the supernatants are then mixed 1:1 with the detection-reagent to a total volume of 100µl for each sample, and incubated for 20min at room temperature. For background determination, only EiMM-medium is measured. In a Genios-reader (TE-CAN, Groedig Software: XFluor4) the luminescence is measured with an integration-time of 1000ms and a signal enhancement of 200.

**Table1: Test substances**

| **Steroid** | **Column** | **isocratic HPLC conditions** |
|---|---|---|
| progesterone | Licrosphere RP18 (Merck, Darmstadt, Germany) | 50 % ACN : 50 % H₂O flow: 1 mL/min; detection at 240 nm |
| testosterone | Licrosphere RP18 (Merck, Darmstadt, Germany) | 50 % ACN : 50 % H₂O flow: 1 mL/min; detection at 240 nm |
| cortisol | Licrosphere RP18 (Merck, Darmstadt, Germany) | 50 % ACN : 50 % H₂O flow: 1 mL/min; detection at 240 nm |
| vitamine D3 | Licrosphere RP 8 (Merck, Darmstadt, Germany) | 90 % methanol: 10 % H₂O flow: 1,5 mL/min; detection at 201 nm |
| DHEA | Licrosphere RP18 (Merck, Darmstadt, Germany) | 50 % ACN : 50 % H₂O flow: 1 mL/min; detection at 201 nm |
| estrone | Licrosphere RP18 (Merck, Darmstadt, Germany) | 50 % ACN: 50 % H₂O flow: 1 mL/min; detection at 201 nm |
| cholesterole | Licrosphere RP 8 (Merck, Darmstadt, Germany) | 90 % methanol : 10 % H₂O flow: 1,5 mL/min; detection at 201 nm |
| 17-alpha-OH progesterone | Licrosphere RP18 (Merck, Darmstadt, Germany) | 50 % ACN : 50 % H₂O flow: 1 mL/min; detection at 240 nm |
| pregnenolone | Licrosphere RP18 (Merck, Darmstadt, Germany) | 50 % ACN : 50 % H₂O flow: 1 mL/min; detection at 240 nm |
| vitamine D2 | Licrosphere RP 8 (Merck, Darmstadt, Germany) | 90 % methanol: 10 % H₂O flow: 1,5 mL/min; detection at 201 nm |
| ebastine | Licrosphere RP18 (Merck, Darmstadt, Germany) | 60 % ACN: 40 % H₂O flow: 1 mL/min; detection at 201 nm |
| deoxycorticosterone | Licrosphere RP18 (Merck, Darmstadt, Germany) | 50 % ACN: 50 % H₂O flow: 1 mL/min; detection at 240 nm |
| 11-deoxcortisole | Licrosphere RP18 (Merck, Darmstadt, Germany) | 50 % ACN: 50 % H₂O flow: 1 mL/min; detection at 240 nm |
| androstendione | Licrosphere RP18 (Merck, Darmstadt, Germany) | 50 % ACN: 50 % H₂O flow: 1 mL/min; detection at 201 nm |

**Table 2: Fatty acids**

| | | |
|---|---|---|
| caprylic acid (C8) | | |
| decanoic acid (C10) | | |
| lauric acid (C12 | | |
| myristic acid (C14) | | |
| palmitic acid (C16) | | |
| stearic acid (C18) | | |
| arachidic acid (C20) | | |
| oleic acid (C18:1) | | |
| linoleic acid (C18:2)= | | |

| | | |
|---|---|---|
| ACN= Acetonitrile | | |

All chemicals were purchased from Sigma-Aldrich except ebastine, which was a generous gift from Almirall SA, Barcelona, Spain.

## Claims

1. Expression system for a functional CYP4 comprising at least one yeast cell population of recombinant *Schizosaccharomyces pombe,* which is transformed with the nucleic acid sequence encoding a CYP4 enzyme.

2. Expression system according to claim 1, wherein CYP4 is the human CYP4Z1, its variants and functional variants.

3. Expression system according to claim 1 or 2, additionally encoding the nucleic acid sequence encoding the human NADPH-dependent cytochrome P450 reductase (CPR).

4. Expression system according to anyone of the previous claims, wherein the nucleic acid sequence encoding the functional CYP4 and CPR are transferred to the yeast cells by one or more integrative or autosomally replicating gene transfer vector.

5. Expression system according to anyone of the previous claims, wherein the nucleic acid sequence encoding the functional CYP4 and CPR are under the control of at least one regulatory element, which is selected from the group consisting of inducible promoters, homologous household promoters and heterologous promoters.

6. Expression system according to anyone of the previous claims, wherein the ratio of the expression of functional CYP4 versus functional CPR is larger than 1:1, preferably 1.1:1, 1,5:1, 2:1, 3:1, 4:1, 5:1, 6:1, 7:1, 8:1, 9:1, 10:1 or even up to 50:1.

7. Use of the expression system according to anyone of the previous claims comprising the recombinant *Schizosaccharomyces pombe,* which is transformed with the nucleic acid sequence encoding a CYP4 and, optionally, the nucleic acid sequence encoding the human NADPH-dependent cytochrome P450 reductase (CPR) for the production of CYP4-dependent metabolites.

8. Method for the detection and/or synthesis of CYP4 dependent comprising the steps of
- culturing under suitable conditions the expression system according to anyone of the previous claims 1 to 6 comprising at least one cell population of recombinant *Schizosaccharomyces pombe,* which is transformed with the nucleic acid sequence encoding a CYP4 and optionally the nucleic acid sequence encoding the human NADPH-dependent cytochrome P450 reductase (CPR), P450 reductase (CPR),
- adding a chemical substance or endogenous substance to the fermentation broth of the cell culture,
- collecting the supernatant, and
- optionally, analysing the supernatant by HPLC or MS, or
- extracting the desired metabolite from the supernatant.

9. Method for the identification of CYP4 modulators comprising the steps of
- culturing under suitable conditions the expression system according to anyone of the claims 1 to 6 comprising at least one cell population of recombinant *Schizosaccharomyces pombe*, which is transformed with the nucleic acid sequence encoding a CYP4 and optionally, the nucleic acid sequence encoding the human NADPH-dependent cytochrome P450 reductase (CPR),
- establishing the bioconversion assay with a reference substance,
- establishing a parallel bioconversion assay with a test substance,
- analysing the supernatant of the bioconversion assay with a reference substance and the bioconversion assay with a test substance for differences in the presents of CYP4 dependent metabolites.

10. Method according to claim 9, wherein the reference substance is luciferin or luciferin-6'-pentafluorobenzyl-ether.

11. Use of the expression system according to anyone of the previous claims 1 to 6 comprising the recombinant *Schizosaccharomyces pombe*, which is transformed with the nucleic acid sequence encoding a CYP4 and optionally the nucleic acid sequence encoding the human NADPH-dependent cytochrome P450 reductase (CPR) for the manufacturing of an enzyme preparation.
